# EUROPEAN PATENT SPECIFICATION

(11) **EP 1 406 613 B1**
(45) Date of publication and mention of the grant of the patent: **28.11.2007**
(21) Application number: 02747327.1
(22) Date of filing: 28.05.2002
(51) Int. Cl.: A61K 31/40, A61K 31/60, A61K 31/621, A61P 1/00, A61P 7/12, A61P 9/00, A61P 9/08, A61P 9/10, A61P 9/12, A61P 25/28, A61P 43/00

(54) **ORGANIC NITRATEDERIVATIVES FOR THE TREATMENT OF ATHEROSCLEROSIS AND VASCULAR DISEASES**
ORGANISCHE NITRAT-DERIVATE ZUR BEHANDLUNG VON ATHEROSKLEROSE UND VASKULÄREN ERKRANKUNGEN
COMPOSES A BASE DE NITRATE ORGANIQUE DESTINES AU TRAITEMENT DE L'ATHEROSCLEROSE ET DES MALADIES VASCULAIRES

(30) Priority: 13.06.2001 IT MI20011240
(43) Date of publication of application: 14.04.2004
(73) Proprietor: NicOx S.A., 06560 Sophia Antipolis - Valbonne (FR)
(72) Inventor: DEL SOLDATO, Piero, I-20052 Monza (IT)
(74) Representative: Barchielli, Giovanna
(86) International application number: PCT/EP2002/005846
(87) International publication number: WO 2002/100400

(56) References cited:
- WO-A-00/51988
- WO-A-00/61537
- WO-A-02/30866
- WO-A-02/30867
- WO-A-95/30641
- WO-A-97/16405
- FIORUCCI, S. ET AL.: "An NO derivative of ursodeoxycholic acid protects against Fas-mediated liver injury by inhibiting caspase activity" PROCEEDINGS OF THE NATIONAL ACADEMY OF SCIENCES, USA, vol. 98, no. 5, 27 March 2001 (2001-03-27), pages 2652-2657, XP001105097
- WALLACE, J.L ET AL.: "Nitric oxide-releasing NSAIDs: GI-safe antithrombotics" IDRUGS, vol. 2, no. 4, 1999, pages 321-326, XP001105153

## Description

The present invention relates to drugs for the prevention and/or the treatment of atherosclerosis and peripheral vascular diseases.

The ideal approach is to operate on the various cell processes, i.e. to prevent the pathological activation of the aforesaid cells, which leads to the onset and to the progress of the pathological process affecting the cardiovascular system.

At present the drugs used for vasculopathies and the used therapeutical approaches inhibit only one cell population, therefore they act only on one phase of the process with only partially satisfactory results.

Statines, the rapamycin and the radiotherapeutic treatment are active only on the smooth musculature but not on the other cell populations. The results obtained with said pharmacological treatments and with the radiotherapy are only partially satisfactory and therefore it is necessary to increase dosages with consequent even serious side effects.

The need was felt to have available drugs allowing to carry out an effective therapeutic treatment of vasculopathies, overcoming the drawbacks associated to therapeutic and surgical treatments at present used, and being effective in inhibiting the pathological activation of different cell populations of the cardiovascular system and, besides, not resulting toxic, in particular at gastric level, and furthermore being usable for prolonged treatments without side effects.

This technical problem has now been solved by the Applicant by using a specific class of drugs. Surprisingly and unexpectedly the Applicant has found that the nitrooxyderivatives of the salicylic acid and derivatives thereof are active in the vasculopathy treatment, acting on the involved cell processes. Said result is surprising since other nitrooxyderivatives, such for example the piroxicam and ketorolac derivatives, have not proved to be active at non toxic doses. The result is still more unexpected if one considers that aspirin acts on the platelets, in a very partially way on monocytes/macrophages, and is inactive on the smooth musculature cells, on leucocytes and on endothelial cells.

An object of the present invention is the use of the compounds having the following formulas for the manufacture of a medicament for the treatment of atherosclerosis and peripheral vascular diseases:
2-(acetyloxy)benzoic acid (4-nitrooxy)butyl ester (X) 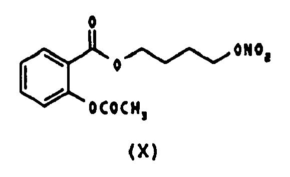
2-(acetyloxy)benzoic acid 3-(nitrooxymethyl)phenyl ester (XI) 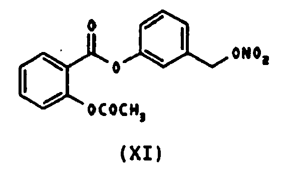
2-(acetyloxy)benzoic acid 4-(nitrooxymethyl)phenyl ester (XII) 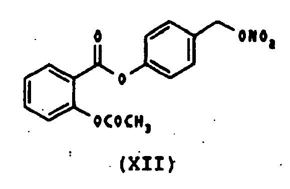
2-(acetyloxy)benzoic acid 2-(nitrooxymethyl)phenyl ester (XIII) 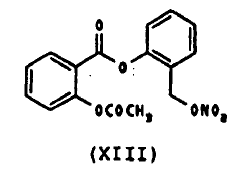
2-(acetyloxy)benzoic acid 2-methoxy-4-[(1E)-3-[4-nitrooxy butoxy]-3-oxo-1-propenyl]phenyl ester (XIV) 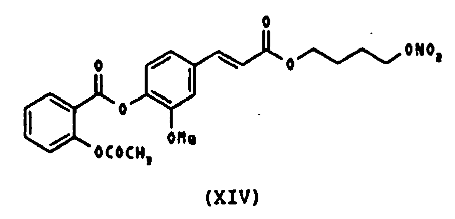
2-(acetyloxy)benzoic acid 6-(nitrooxymethyl)-2-methyl pyridinyl hydrochloride ester (XV) 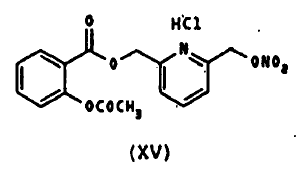
2-hyroxy-benzoic acid, 3-(nitrooxymethyl)phenyl ester (XVI) 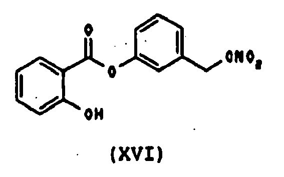
2-(hydroxy)benzoic acid 4-(nitrooxymethyl)phenyl ester (XVII) 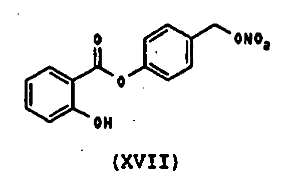
2-(hydroxy)benzoic acid 4-(nitrooxy)butyl ester (XVIII) 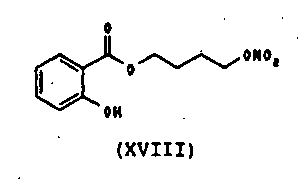
2-(hydroxy)benzoic acid 2-(nitrooxymethyl)phenyl ester (XIX) 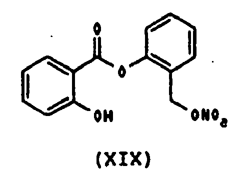
2-(hydroxy)benzoic acid 2-methoxy-4-[(1E)-3-[4-nitrooxy butoxyl]-3-oxo-1-propenyl]phenyl ester (XX) 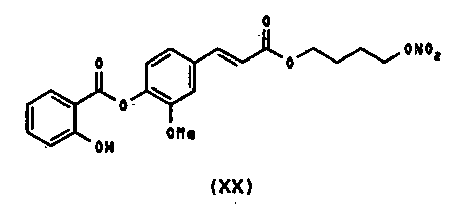
N-acetylcysteine, 4-nitrooxybutyl ester, 2-acetyloxy-benzoate (XXI) 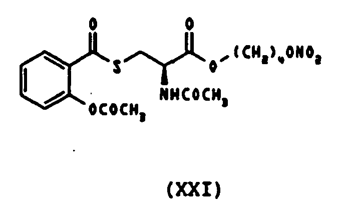
2-hydroxybenzoic acid, 6-(nitrooxymethyl)-2-methyl pyridinyl hydrochloride ester (XXII) 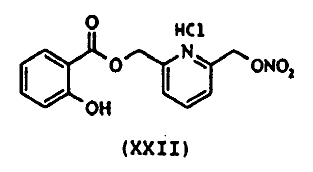
2-hydroxybenzoic acid, 5-(nitrooxymethyl)-2-methyl pyridinyl hydrochloride ester (XXIII) 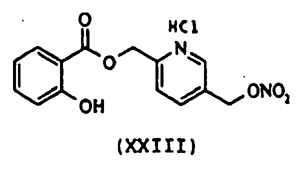
2-hydroxybenzoic acid, 3-(nitrooxymethyl)-2-methyl pyridinyl hydrochloride ester (XXIV) 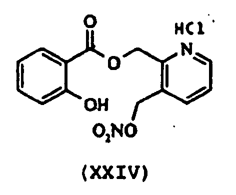
2-(acetyloxy)benzoic acid, 5-(nitrooxymethyl)-2-methylpyridinyl hydrochloride ester (XXV) 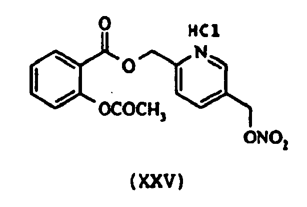
2-hydroxybenzoic acid, 6-(nitrooxymethyl)-2-methyl pyridinyl hydrochloride ester (XXVI) 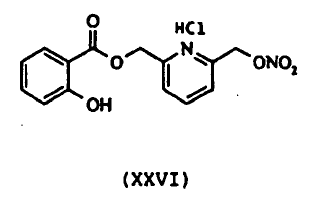
2-hydroxybenzoic acid, 5-(nitrooxymethyl)-2-methyl pyridinyl hydrochloride ester (XXVII) 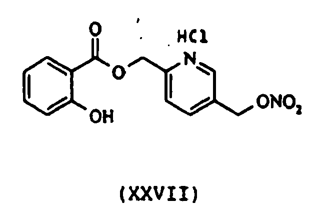
2-hydroxybenzoic acid, 3-(nitrooxymethyl)-2-methyl pyridinyl hydrochloride ester (XXVIII) 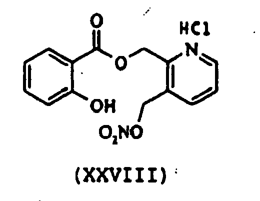
2-(acetyloxy)benzoic acid, 5-(nitrooxymethyl)-2-methylpyridinyl hydrochloride ester (XXIX) 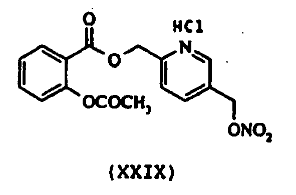
2-(acetyloxy)benzoic acid, 3-(nitrooxymethyl)-2-methylpyridinyl hydrochloride ester (XXX) 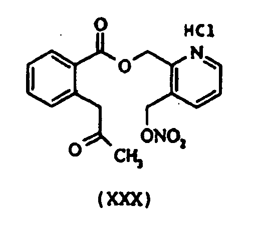
2-(acetyloxy)benzoic acid, 3-(nitrooxymethyl)-2-methylpyridinyl hydrochloride ester (XXXI) 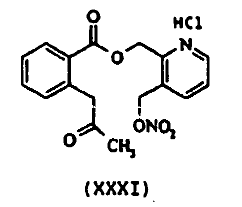
The compounds of the invention are generally obtained by methods known in the art, see for example patent applications WO 00/61537 for compounds (XVI), (XX) and (XXI), WO 00/51988 for compounds (XV) and (XXII) to (XXXI) and WO 95/30641 for compounds (X) to (XIII) and (XVI) to (XIX), in the name of the applicant.

The nitrooxyderivatives of the salicylic acid can also be synthesized starting from the corresponding nitrooxyderivatives of the acetylsalicylic acid, prepared according to the methods described in the above patent applications, by selective hydrolysis of the acetyl group. See the Examples, in particular Example 15, of WO 02/30866 in the name of the Applicant.

When the compounds of formula (I) usable in the present invention have one or more chiral centres, they can be in a racemic form or as mixtures of diastereoisomers, as single enantiomers or single diastereoisomers; when they show geometric asymmetry, the compounds in the cis or trans form can be used.

When in the molecule of the compounds of formula (I) a salifiable functional group is present, for example an aminic or heterocyclic nitrogen, it is possible to use the corresponding salts of the above compounds, obtainable by raction in organic solvent such as for example acetonitrile, tetrahydrofuran, with an equimolar amount of the corresponding organic or inorganic acid.

Examples of usable organic acids are the following: oxalic, tartaric, maleic, succinic, citric acid.

Examples of usable inorganic acids are the following: nitric, hydrochloric, sulphuric, phosphoric acid. Nitric and hydrocloric acids are preferred.

By using the products of the invention, the vasculopathy is significantly reduced and in particular the restenosis process which can arise in people subjected to angioplasty and in particular in those more at risk such as old people, diabetic, hyperlipidemic people.

The therapeutic use of the compounds described in the preent invention results advantageous, as said, since these compounds are able to act both on the duct (endothelial and vasal smooth musculature cells) and on the haematic cells (platelets, leucocytes) and haematic factors.

The compounds of the invention are formulated in the corresponding pharmaceutical compositions for parenteral, oral use according to the techniques well known in the prior art, together with the usual excipients; see for example the volume "Remington's Pharmaceutical Sciences" 15th Ed.

The amount on a molar basis of the active principle in said formulations is equal to or lower than the maximum posology indicated for the precursor drugs. Also higher doses can be used in consideration of their very good tolerability. The daily doses of the precursor drugs can be found in th publications of the prior art, such as for example in "Physician's Desk Reference".

The following Examples illustrate the invention.

### EXAMPLE F3

Evaluation of the vascular damage in animals treated with NO-ASA and Aspirin.

In this experiment SP-SHR rats were used.

Three groups, each forced by 12 SP-SHR rats, 8 weeks old at the beginning of the experiment, received respectively for 6 weeks, together with the daily diet Aspirin (54 mg/kg) NO-ASA (30 mg/kg) (two groups); the control group (third group) received only the diet.

At the end of the treatment the animals were sacrificed by decapitation and the carotids were isolated. The ducts were opened and washed with cold sterile buffer phosphate (PBS) containing EDTA (2mM) and maintained in cold PBS (cooled in ice bath) containing 2,[6]-di-tert-butyl-p-cresol (50 µM), aprotin (0.001%), EDTA (50mM) and chloramphenicol (0.008%). The arteries were fixed with formalin (10%), then soaked in paraffin and then dissected. An aliquot of the obtained sections was incubated with MCA2 antibodies, which are directed against specific epitopes for oxidized LDL.

The obtained results are reported in Table 3. The data were calculated by considering the number of the sections, positive at the immunohistochemical test with MDA2 antibodies, detected in the groups of the treated animals and in the control group, respectively. The results are expressed as percentage of reduction of the oxidized LDL (low density lipoprotein) presence in the vascular wall taking as 0 the LDL value measured in the control group.

The oxidized lipoprotein content was found to be correlated with the severity of the disease and the mortality incidence in the treated. This datum is therefore of particular importance.

The reduction of oxidized LDL is an index of the vasal protection from thrombogenic damage which is the triggering factor of cerebral infarct.

**Table 3**

| Evaluation of the vascular damage in carotids of rats treated with NO-ASA and Aspirin, determined as reduction % of the presence of oxidized LDL in the vascular wall | | |
|---|---|---|
| Treatment | Dose (mg/kg) | reduction % of the presence of oxidized LDL |
| Controls | - | 0 |
| NO-ASA | 30 | 74 |
| Aspirin | 54 | 19 |

## Claims

1. The use of one of the following compounds for the manufacture of a medicament for the treatment of atherosclerosis and peripheral vascular diseases:
2-(acetyloxy)benzoic acid (4-nitrooxy)butyl ester (X) 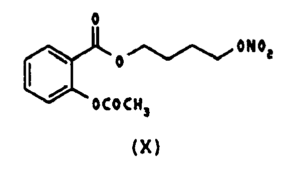
2-(acetyloxy)benzoic acid 3-(nitrooxymethyl)phenyl ester (XI) 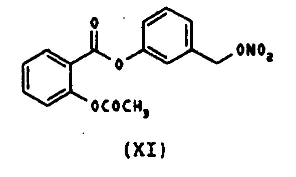
2-(acetyloxy)benzoic acid 4-(nitrooxymethyl)phenyl ester (XII) 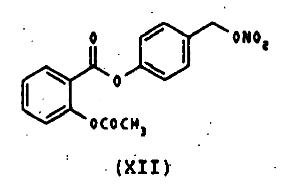
2-(acetyloxy)benzoic acid 2-(nitrooxymethyl)phenyl ester (XIII) 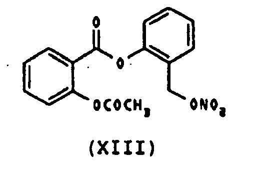
2-(acetyloxy)benzoic acid 2-methoxy-9-[(1E)-3-[4-nitrooxy butoxy]-3-oxo-1-propenyl]phenyl ester (XIV) 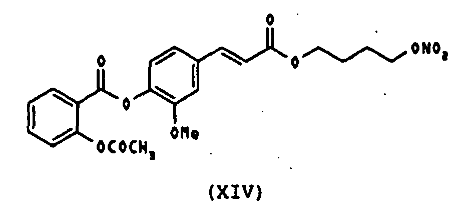
2-(acetyloxy)benzoic acid 6-(nitrooxymethyl)-2-methyl pyridinyl hydrochloride ester (XV) 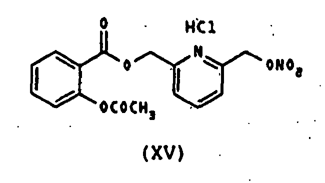
2-hyroxy-benzoic acid, 3-(nitrooxymethyl)phenyl ester (XVI) 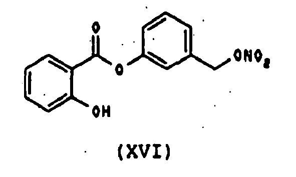
2-(hydroxy)benzoic acid 4-(nitrooxymethyl)phenyl ester (XVII) 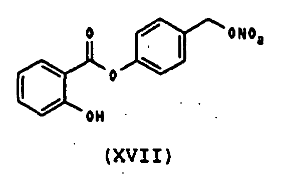
2-(hydroxy)benzoic acid 4-(nitrooxy)butyl ester (XVIII) 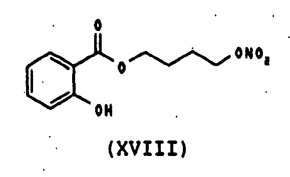
2-(hydroxy)benzoic acid 2-(nitrooxymethyl)phenyl ester (XIX) 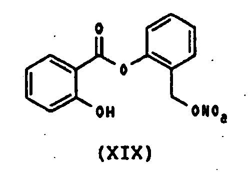
2-(hydroxy)benzoic acid 2-methoxy-4-[(lE)-3-[4-nitrooxy butoxyl]-3-oxo-1-propenyl]phenyl ester (XX) 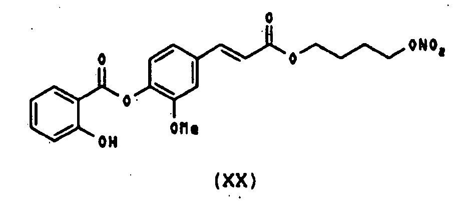
N-acetylcysteine, 4-nitrooxybutyl ester, 2-acetyloxy-benzoate (XXI) 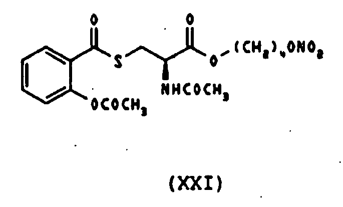
2-hydroxybenzoic acid, 6-(nitrooxymethyl)-2-methyl pyridinyl hydrochloride ester (XXII) 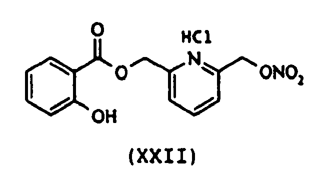
2-hydroxybenzoic acid, 5-(nitrooxymethyl)-2-methyl pyridinyl hydrochloride ester (XXIII) 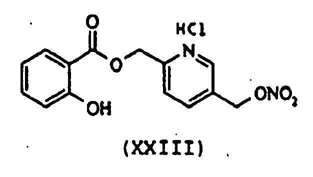
2-hydroxybenzoic acid, 3-(nitrooxymethyl)-2-methyl pyridinyl hydrochloride ester (XXIV) 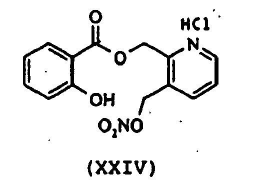
2-(acetyloxy)benzoic acid, 5-(nitrooxymethyl)-2-methylpyridinyl hydrochloride ester (XXV) 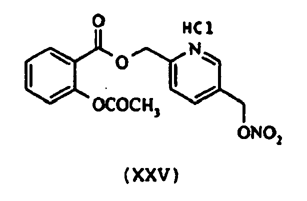
2-hydroxybenzoic acid, 6-(nitrooxymethyl)-2-methyl pyridinyl hydrochloride ester (XXVI) 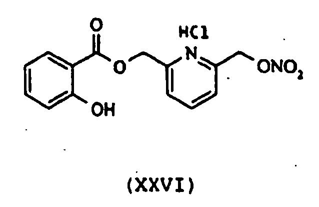
2-hydroxybenzoic acid, 5-(nitrooxymethyl)-2-methyl pyridinyl hydrochloride ester (XXVII) 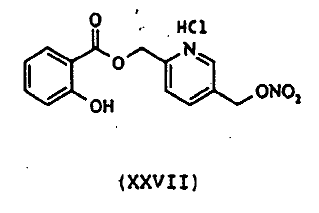
2-hydroxybenzoic acid, 3-(nitrooxymethyl)-2-methyl pyridinyl hydrochloride ester (XXVIII) 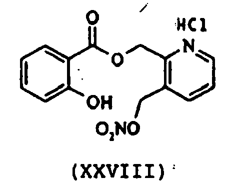
2-(acetyloxy)benzoic acid, 5-(nitrooxymethyl)-2-methylpyridinyl hydrochloride ester (XXIX) 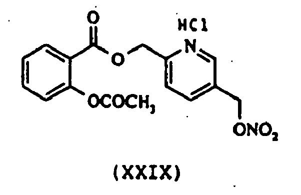
2-(acetyloxy)benzoic acid, 3-(nitrooxymethyl)-2-methylpyridinyl hydrochloride ester (XXX) 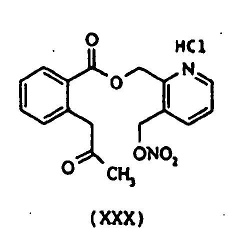
2-(acetyloxy)benzoic acid, 3-(nitrooxymethyl)-2-methylpyridinyl hydrochloride ester (XXXI)

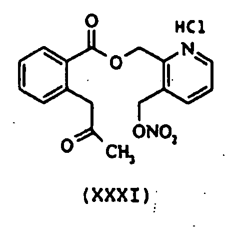

2. The use according to claim 1, wherein the medicament is formulated for parenteral, oral or topical use.

## Patentansprüche

1. Verwendung einer der folgenden Verbindungen zur Herstellung eines Medikaments zur Behandlung von Atherosclerose und peripheren vasculären Erkrankungen:
2-(Acetyloxy)benoesäure (4-nitrooxy)butylester (X) 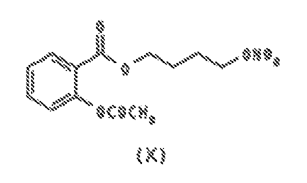
2-(Acetyloxy)benzoesäure 3-(nitrooxymethyl)phenylester (XI) 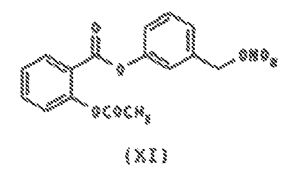
2-(Acetyloxy)benzoesäure 4-(nitrooxymethyl)phenylester (XII) 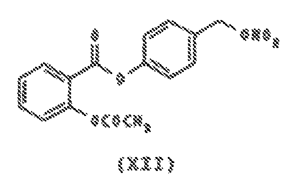
2-(Acetyloxy)benzoesäure 2-(nitrooxymethyl)phenylester (XIII) 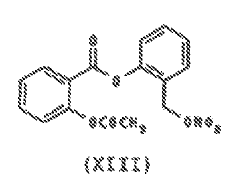
2-(Acetyloxy)benzoesäure 2-methoxy-4-[(1E)-3-[4-nitroxybutoxyl]-3-oxo-1-propenyl]phenylester (XIV) 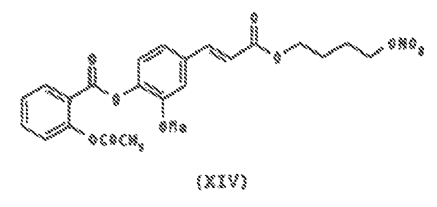
2-(Acetyloxy)benzoesäure 6-(nitrooxymethyl)-2-methylpyridinylhydrochloridester (XV) 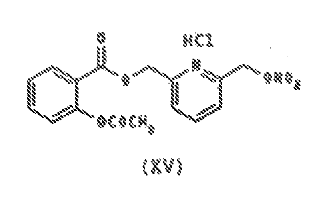
2-Hydroxy-benzoesäure, 3-(nitrooxymethyl)phenylester (XVI) 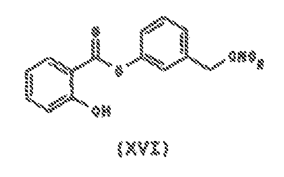
2-(Hydroxy)benzoesäure 4-(nitrooxymethyl)phenylester (XVII) 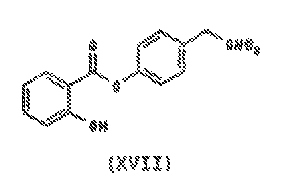
2-(Hydroxy)benzoesäure 4-(nitrooxy)butylester (XVIII) 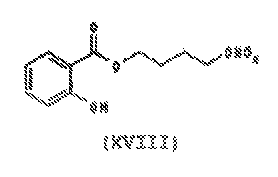
2-(Hydroxy)benzoesäure 2-(nitrooxymethyl)phenylester (XIX) 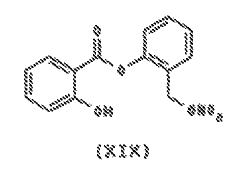
2-(Hydroxy)benzoesäure 2-methoxy-4-[(1E)-3-[4-nitrooxybutoxyl]-3-oxo-1-propenyl]phenylester (XX) 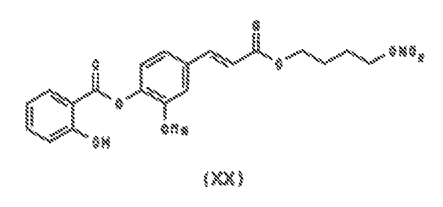
N-Acetylcystein, 4-nitrooxybutylester, 2-acetyloxybenzoat (XXI) 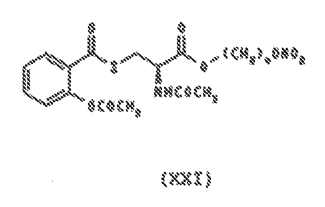
2-Hydroxybenzoesäure, 6-(nitrooxymethyl)-2-methylpyridinylhydrochloridester (XXII) 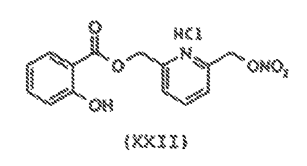
2-Hydroxybenzoesäure, 5-(nitrooxymethyl)-2-methylpyridinylhydrochloridester (XXIII) 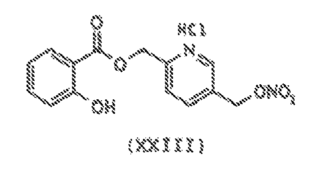
2-Hydroxybenzoesäure, 3-(nitrooxymethyl)-2-methylpyridinylhydrochloridester (XXIV) 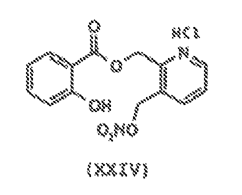
2-(Acetyloxy)benzoesäure, 5-(nitrooxymethyl)-2-methylpyridinylhydrochloridester (XXV) 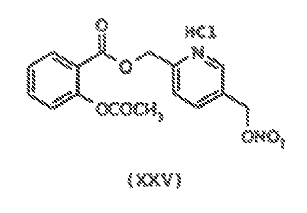
2-Hydroxybenzoesäure, 6-(nitrooxymethyl)-2-methylpyridinylhydrochloridester (XXVI) 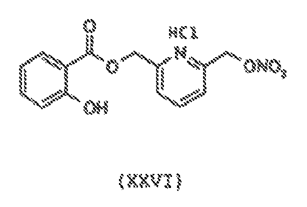
2-Hydroxybenzoesäure, 5-(nitrooxymethyl)-2-methylpyridinylhydrochloridester (XXVII) 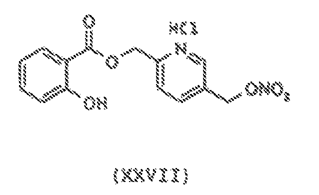
2-Hydroxybenzoesäure, 3-(nitrooxymethyl)-2-methylpyridinylhydrochloridester (XXVIII) 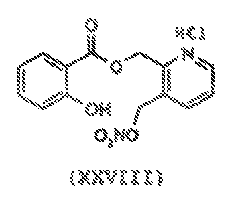
2-(Acetyloxy)benzoesäure, 5-(nitrooxymethyl)-2-methylpyridinylhydrochloridester (XXIX) 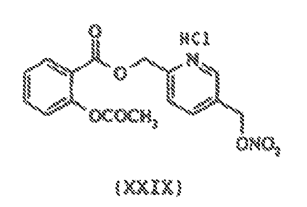
2-(Acetyloxy)benzoesäure, 3-(nitrooxymethyl)-2-methylpyridinylhydrochloridester (XXX) 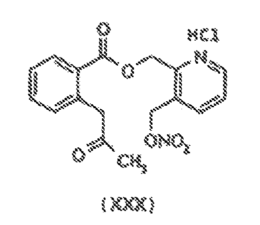
2-(Acetyloxy)benzoesäure, 3-(nitrooxymethyl)-2-methylpyridinylhydrochloridester (XXXI)

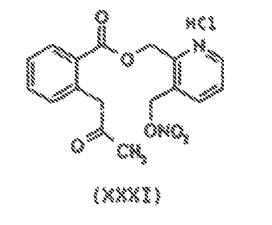

2. Verwendung gemäß Anspruch 1, wobei das Medikament für die parenterale, orale oder topische Verwendung formuliert ist.

## Revendications

1. Utilisation de l'un des composés suivants pour la fabrication d'un médicament destiné au traitement de l'athérosclérose et de maladies vasculaires périphériques:
Ester (4-nitrooxy)butylique d'acide 2-(acêtyloxy)benzoïque (X) 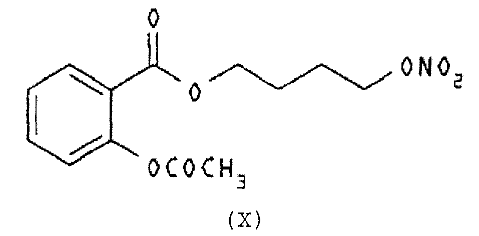
Ester 3-(nitrooxyméthyl)phénylique d'acide 2-(acétyloxy)-benzoïque (XI) 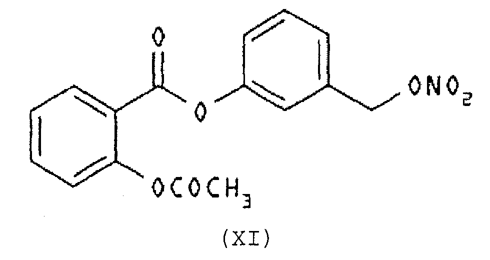
Ester 4-(nitrooxyméthyl)phénylique d'acide 2-(acétyloxy)-benzoïque (XII) 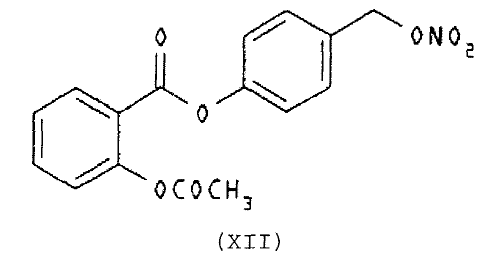
Ester 2-(nitrooxyméthyl)phénylique d'acide 2-(acétyloxy)-benzoïque (XIII) 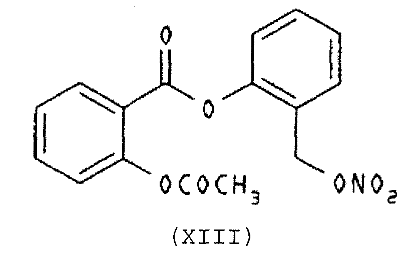
Ester 2-(méthoxy)-4-[(1E)-3-[4-nitrooxybutoxy]-3-oxo-1-propényl]phénylique d'acide 2-(acétyloxy)benzoïque (XIV) 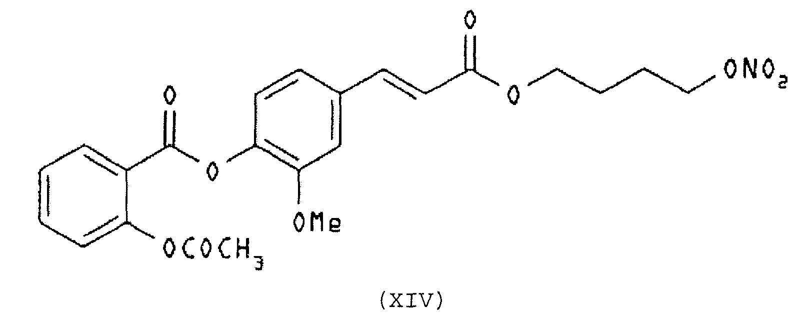
Ester du chlorhydrate de 6-(nitrooxyméthyl)-2-méthylpyridinyle avec l'acide 2-(acétyloxy)benzoïque (XV) 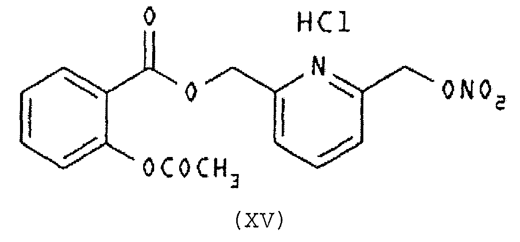
Ester 3-(nitrooxyméthyl)phénylique d'acide 2-(hydroxy)-benzoïque (XVI) 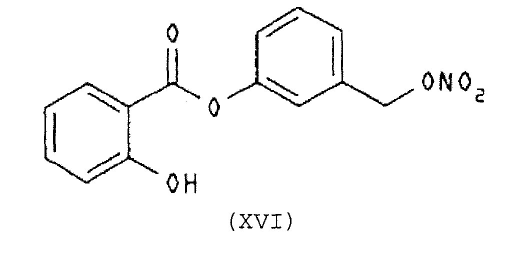
Ester 4-(nitrooxyméthyl)phénylique d'acide 2-(hydroxy)-benzoïque (XVII) 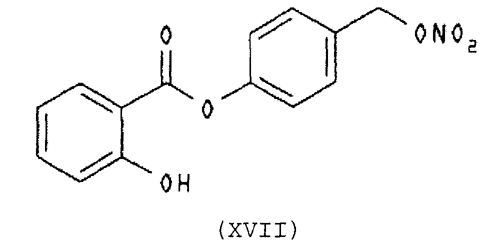
Ester 4-(nitrooxy)butylique d'acide 2-(hydroxy)benzoïque (XVIII) 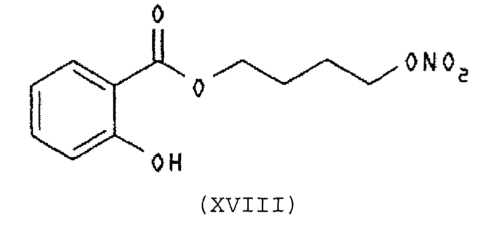
Ester 2-(nitrooxyméthyl)phénylique d'acide 2-(hydroxy)-benzoïque (XIX) 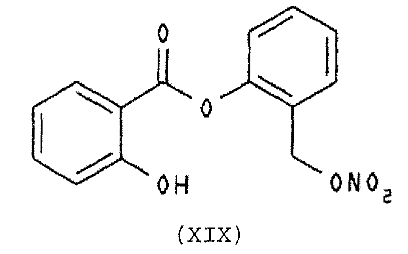
Ester 2-(méthoxy)-4-[(lE)-3-[4-nitrooxybutoxy]-3-oxo-1-propényl]phénylique d'acide 2-(hydroxy)benzoïque (XX) 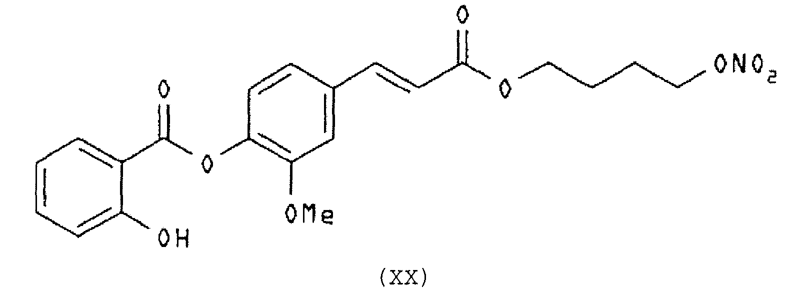
2-acétyloxybenzoate d'ester 4-nitrooxybutylique de la N-acétylcystéine (XXI) 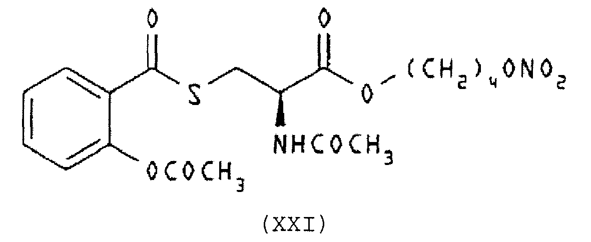
Ester du chlorhydrate de 6-(nitrooxyméthyl)-2-méthylpyridinyle avec l'acide 2-hydroxybenzoïque (XXII) 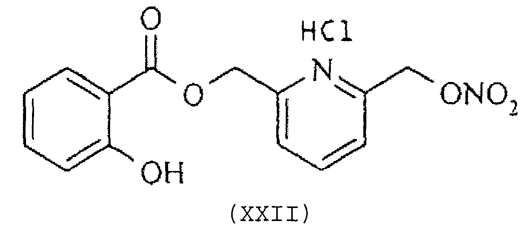
Ester du chlorhydrate de 5-(nitrooxyméthyl)-2-méthylpyridinyle avec l'acide 2-hydroxybenzoïque (XXIII) 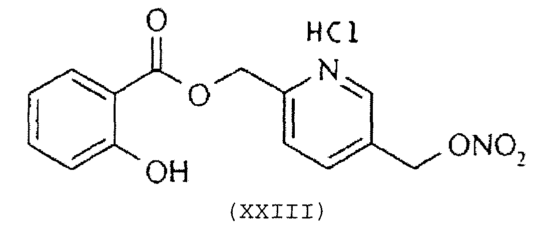
Ester du chlorhydrate 3-(nitrooxyméthyl)-2-méthylpyridinyle avec l'acide 2-hydroxybenzoïque (XXIV) 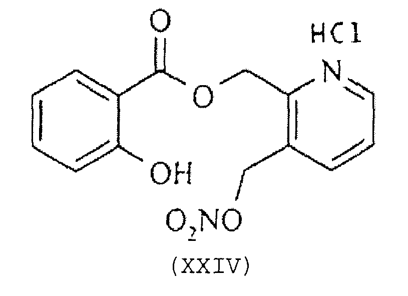
Ester du chlorhydrate de 5-(nitrooxyméthyl)-2-méthylpyridinyle avec l'acide 2-(acétyloxy)benzoïque (XXV) 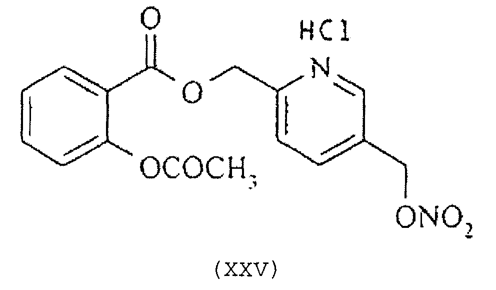
Ester du chlorhydrate de 6-(nitrooxyméthyl)-2-méthylpyridinyle avec l'acide 2-hydroxybenzoïque (XXVI) 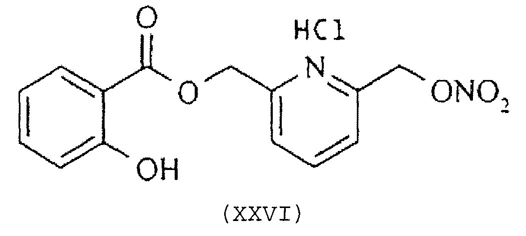
Ester du chlorhydrate de 5-(nitrooxyméthyl)-2-méthylpyridinyle avec l'acide 2-hydroxybenzoïque (XXVII) 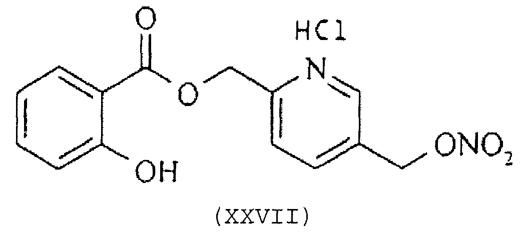
Ester du chlorhydrate de 3-(nitrooxyméthyl)-2-méthylpyridinyle avec l'acide 2-hydroxybenzoïque (XXVIII) 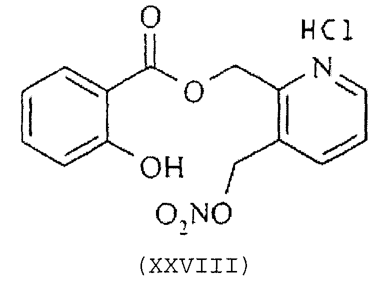
Ester du chlorhydrate de 5-(nitrooxyméthyl)-2-méthylpyridinyle avec l'acide 2-(acétyloxy)benzoïque (XXIX) 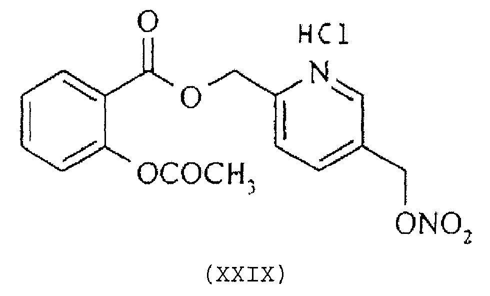
Ester du chlorhydrate de 3-(nitrooxyméthyl)-2-méthylpyridinyle avec l'acide 2-(acétyloxy)benzoïque (XXX) 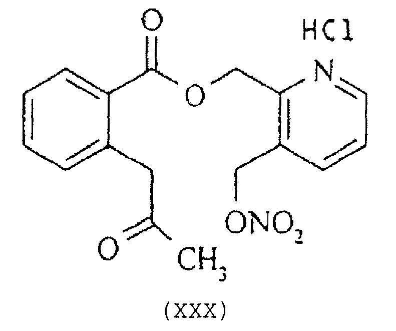
Ester du chlorhydrate de 3-(nitrooxyméthyl)-2-méthylpyridinyle avec l'acide 2-(acétyloxy)benzoïque (XXXI)

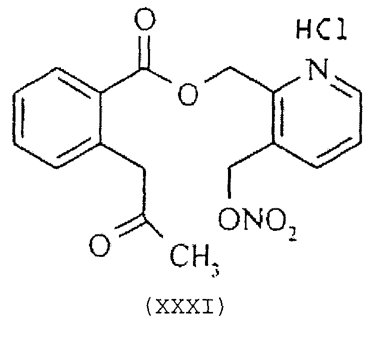

2. Utilisation selon la revendication 1, dans laquelle le médicament est formulé pour une utilisation par voie parentérale, orale ou topique.
